# EUROPÄISCHE PATENTSCHRIFT

(11) **EP 1 150 643 B1**
(45) Veröffentlichungstag und Bekanntmachung des Hinweises auf die Patenterteilung: **15.01.2003**
(21) Anmeldenummer: 00908994.7
(22) Anmeldetag: 07.02.2000
(51) Int. Cl.: A61K 7/00

(54) **MIKROEMULSION, ENTHALTEND ALKANOLAMMONIUM-SALZE DER ALKYLSULFATE UND/ODER ALKYLPOLYALKYLENGLYKOLETHERSULFATE**
MICROEMULSION CONTAINING ALKANO LAMMONIUM SALTS OF FATTY ALCOHOL SULFATES AND/OR ALKYLPOLY ALKYLENE GLYKOL ETHER SULFATES
MICROEMULSION CONTENANT LES SELS ALKANO LAMMONIUM DE SULFATES D'ALKYLE ET/OU DE SULFATES D'ALKYLPOLY ALKYENEGLYCOLETHER

(30) Priorität: 08.02.1999 DE 19904847
(43) Veröffentlichungstag der Anmeldung: 07.11.2001
(73) Patentinhaber: SASOL Germany GmbH, 22297 Hamburg (DE)
(72) Erfinder: BROCK, Michael, D-46514 Schermbeck (DE); DIESVELD-KOLLER, Sabine, D-48712 Gescher (DE); KOBERSTEIN, Eva-Maria, D-45657 Recklinghausen (DE); MICHEL, Ursula, D-46282 Dorsten (DE); NAPIERALA, Heinz, D-45699 Herten (DE); STOLZ, Martin, D-48249 Dülmen (DE)
(74) Vertreter: Schupfner, Georg U.
(86) Internationale Anmeldenummer: DE0000357
(87) Internationale Veröffentlichungsnummer: WO00047166

(56) Entgegenhaltungen:
- EP-A- 0 226 337
- EP-A- 0 387 647
- EP-A- 0 867 176
- EP-A- 0 878 189
- WO-A-95/12379
- WO-A-99/09943
- DE-A- 19 528 394
- DE-A- 19 755 488

## Beschreibung

Die Erfindung betrifft Mikroemulsionen enthaltend Alkanolammonium-Salze der Alkylsulfate und/oder Alkylpolyalkylenglykolethersulfate und deren Verwendung im kosmetischen und/oder medizinisch-dermatologischen Bereich.

Der Einsatz von Mikroemulsionen findet insbesondere in Anwendungsgebieten, in denen der gleichzeitige Einsatz einer wäßrigen Phase und einer Ölkomponente gewünscht wird, zunehmendes Interesse. Einen Überblick der Einsatzgebiete von Mikroemulsionen liefert z.B. V. Chhabra et al. in Tensid Surf. Det. 34(1997),156-168. In dieser Veröffentlichung wird z.B. der Einsatz von Mikroemulsionen in Reinigungsmitteln beschrieben.

Auch der Einsatz von Emulsionen im kosmetischen und medizinisch-dermatologischen Bereich ist interessant. Zusammensetzungen, die als Körperreinigungs- und gleichzeitig als Körperpflegemittel Verwendung finden sollen, müssen verschiedensten Ansprüchen genügen. Sie sollen z.B. die reinigenden Eigenschaften einer wäßrigen Tensidformulierung mit den pflegenden Eigenschaften einer fettenden Ölkomponente vereinen. Mittel, die zugleich zur Körperpflege und Körperreinigung verwendet werden, sind hinsichtlich ihrer Zusammensetzung mit herkömmlichen Reinigungsmitteln, wie sie für Fußböden, Textilien oder Geschirr eingesetzt werden, nicht vergleichbar.

Die Reinigung der Haut und des Haars wird in der Regel von Tensiden übernommen. Je nach Art der verwendeten Tenside bewirken diese eine mehr oder weniger stark ausgeprägte Quellung und nachfolgendes Austrocknen der Hornschicht der Haut, wodurch der Schutzmechanismus der Hautoberfläche gestört wird. Daher werden den bekannten Mitteln zur Reinigung der Haut in zunehmenden Maß hautpflegende Komponenten zur Hautregeneration zugesetzt. Weiterhin können diesen Mitteln auch stoffwechselanregende Komponenten zugesetzt sein, die das Allgemeinbefinden steigern. Dies gilt vor allem für Schaumbadeöle, die seit kurzem auf dem Markt erhältlich sind. Neben anderen Wirkstoffen enthalten diese im wesentlichen wasserfreien Produkte Tenside zur Hautreinigung sowie hohe Anteile an Ölen für die Hautpflege. Nachteil der Schaumbadeöle ist, daß ein Großteil der darin enthaltenen Öle bei der Anwendung auf der Wasseroberfläche des Wanneninhalts verbleiben und somit aufgrund des geringen Kontakts mit der Haut nur in einem eingeschränkten Maß als pflegende Komponente zur Verfügung stehen. Die Öle sind zum überwiegenden Teil ungenutzt Bestandteil des Abwassers.

Ähnliches gilt für Ölduschbäder, wie sie in der US 5,653,988 bzw. in der DE 197 12 678-A1 beschrieben sind. Es werden dort im wesentlichen wasserfreie und tensidhaltige kosmetische oder dermatologische Duschöle, die mindestens 45 % bzw. 30 % einer oder mehrerer Ölkomponenten enthalten, offenbart. Ein Großteil der Ölkomponenten gelangt auch hier ohne Wirkung in das Abwasser, da bei der Anwendung während des Duschens eine hohe Überdosierung des Ölanteils vorliegt.

Ein weiterer Nachteil der Schaumbadeöle und Duschöle ist der hohe Preis der darin enthaltenen Rohstoffe, da diese wenig bzw. kein Wasser enthalten. Es hat daher in der Vergangenheit nicht an Versuchen gefehlt, den Anteil des Öls bei Erhöhung des Wassergehalts unter Beibehaltung des Schäumvermögens und Verbesserung des Leistung/Preis-Verhältnisses zu verringern.

So offenbart die Schrift US 4,371,548 schäumende und tensidhaltige Bade- und Duschzubereitungen mit einem Ölanteil von 20 bis 60 % und optional einem Wassergehalt von maximal 15 %. Diese Zubereitungen weisen Nachteile auf und sind z.B. noch immer zu ungünstig im Leistung/Preis-Verhältnis, da der Wassergehalt unter Beibehaltung der geforderten Eigenschaften (gute Hautreinigung bei guter Schaumentwicklung und hohe Hautpflegewirkung) zu gering ist.

Die Art der Ölkomponente und deren Anteil in der späteren Formulierung sind ebenso wie der Anteil der wäßrigen Phase und deren Zusammensetzung häufig durch die Erfordernisse des Anwendungsbereiches vorgegeben. Während dem Fachmann die Auswahl eines geeigneten Tensides zur Herstellung einer Makroemulsion aus der breiten Palette der am Markt befindlichen Tenside keine Schwierigkeit bereitet, stellt die Herstellung einer Mikroemulsion deutlich größere Probleme dar. Grund hierfür ist, daß die Phasengebiete einer Öl/Wasser/Tensid-Mischung, bei denen sich eine Makroemulsion ausbildet, deutlich größer sind, als die Phasengebiete, bei denen eine Mikroemulsion vorliegt.

Es hat in der Vergangenheit nicht an Versuchen gefehlt, Zubereitungen herzustellen, die Körperreinigungsmittel und Körperpflegemittel zugleich sind. Als Körperreinigungs- und Körperpflegemittel sind in diesem Zusammenhang Produkte zur Reinigung und Pflege der Haut und/oder des Haars während des Duschens, Waschens und Badens gemeint.

Für den Einsatz der erfindungsgemäßen Zusammensetzungen im kosmetischen und medizinisch-dermatologischen Bereich hat sich überraschend gezeigt, daß man durch die erfindungsgemäßen Mikroemulsionen die reinigenden Eigenschaften einer wäßrigen Tensidformulierung mit den pflegenden Eigenschaften einer fettenden Ölkomponente vereinen kann und durch die feinere Verteilung der Öltröpfchen in der Mikroemulsion auch eine bessere Verteilung der pflegenden Ölkomponente auf der Haut erzielen kann.

Bei der Formulierung von kosmetischen oder medizinisch-dermatologischen Zubereitungen kommt erschwerend hinzu, daß die zur Bildung der Mikroemulsionen eingesetzten Tenside eine gute Hautverträglichkeit aufweisen sollten, und sich daher die Auswahl eines geeigneten Tensides zusätzlich erschwert.

In der Literatur sind vorwiegend Mikroemulsionen beschrieben, bei denen nichtionische Tenside, wie z.B. die Alkoholoxethylate, eingesetzt werden. Solche Tenside haben in Zubereitungen, die für die Verwendung auf der menschlichen Haut vorgesehen sind, den Nachteil, daß sie zu stark entfettend wirken. Bei anionischen Tensiden ist man häufig auf den Einsatz von Coemulgatoren angewiesen, um Mikroemulsionen auszubilden.

Mikroemulsionen enthaltend Alkylpolyalkylenglykolethersulfate oder Alkylsulfate sind an sich bekannt. In der DE 35 34 733 A1 werden schäumende Tensidzubereitungen mit klar solubilisierten wasserunlöslichen Ölkomponenten, die nach allgemeinem Verständnis als Mikroemulsionen zu bezeichnen sind, offenbart. In dieser Schrift wird ausdrücklich darauf hingewiesen, daß auf den Zusatz niederer Alkohole oder Alkylglykole mit C₁- bis C₄- Alkylgruppen zu verzichten ist. In der EP 0 638 634 A2 werden Tensid-Mikroemulsionen als Allzweckreiniger offenbart, die zwingend für kosmetische Anwendungen wenig geeignete Tenside vom Sulfonat-Typ enthalten.

Aufgabe der vorliegenden Erfindung war es, Abhilfe von den oben geschilderten Problemen bei der Formulierung von kosmetischen und medizinisch-dermatologischen Mikroemulsionen zu schaffen und Tenside bereitzustellen, mit denen Mikroemulsionen mit einem Anteil der Ölkomponente von maximal 20 % und einem hohen Wasseranteil bei einer möglichst geringen Tensidmenge hergestellt werden können.

Überraschenderweise wurde gefunden, daß kosmetische und medizinischdennatologische Zubereitungen, insbesondere Bade- und Duschzubereitungen, sowie Flüssigseifen und Shampoos mit dem geforderten Anforderungsprofil hergestellt werden können, die mit geringerem Ölgehalt und höherem Wasseranteil als Mikroemulsionen vorliegen.

Gegenstand der vorliegenden Erfindung sind Mikroemulsionen enthaltend:
(A) 0,5 bis 70 Gew.-% Alkanolammonium-Salze der Alkylsulfate und/oder Alkylpolyalkylenglykolethersulfate der folgenden Struktur

   R¹ -O-(CₚH₂ₚO)ₘ-SO₃⁻HN⁺R² R³ R⁴ ,

   worin
   - R¹ =: ein C₈- bis C₂₀- Kohlenwasserstoffrest ist,
   - p =: eine ganze Zahl von 2 bis 5 ist, wobei p für jedes m verschieden sein kann,
   - R² =: H, ein C₁- bis C₆- Alkyl oder Hydroxyisopropyl,
   - R³ =: H, ein C₁- bis C₆- Alkyl oder Hydroxyisopropyl,
   - R⁴ =: Hydroxyisopropyl und
   - m =: eine ganze Zahl von 0 bis 7 ist,
(B) 20 bis 95 Gew.-% Wasser,
(C) 0,1 bis 20 Gew.-% einer oder mehrerer Ölkomponenten,
(D) 0,1 bis 20 Gew.-%, vorzugsweise 0,1 bis 15 Gew.-%, einen oder mehrere einoder mehrwertige, vorzugsweise ein-, zwei- oder dreiwertige, C₂- bis C₂₄- Alkohole, vorzugsweise C₂- bis C₆-Alkohole.

Die erfindungsgemäßen Mikroemulsionen können weiterhin zumindest eine der folgenden Komponenten enthalten:
(E) 0 bis 20 Gew.-%, vorzugsweise 3 bis 15 Gew.-%, ein oder mehrere weitere Tenside,
(F) 0 bis 20 Gew.-%, vorzugsweise 1 bis 12 Gew.-%, oder auch 3 bis 12 Gew.-%, ein oder mehrere Elektrolyte und
(G) 0 bis 10 Gew.-%, vorzugsweise 0,1 bis 8 Gew.-% ein oder mehrere Additive.

Die Mikroemulsionen weisen besonders vorteilhaft die oben genannten Komponenten in den unten angegebenen Konzentrationen unabhängig voneinander auf:
(A) zu 2 bis 60 Gew.-%, vorzugsweise 20 bis 40 Gew.-%,
(B) zu 30 bis 80 Gew.-%, vorzugsweise 40 bis 60 Gew.-%,
(C) zu 0,5 bis 15 Gew.-%, vorzugsweise 4 bis 10 Gew.-%,
(D) zu 0,1 bis 9 Gew.-%, vorzugsweise 0,5 bis 9 Gew.-%,
(E) 0 bis 20 Gew.-%, vorzugsweise 3 bis 15 Gew.-%, weitere Tenside,
(F) 0 bis 20 Gew.-%, vorzugsweise 1 bis 12 Gew.-%, Elektrolyte und
(G) 0 bis 10 Gew.-%, vorzugsweise 0,1 bis 8 Gew.-% Additive,
worin weiterhin besonders vorteilhaft:
(E) als ein weiteres Tensid ein mit Ethylenoxid und/oder Propylenoxid oxalkyliertes und anschließend mit C₆- bis C₂₂- Fettsäuren teilweise oder ganz verestertes Triglycerid ist und/oder
(G) als zumindest ein Additiv ein Poly(C₂- bis C₄-)alkylenglykol mit einem Molgewicht von bis zu 1500 g/mol ist.

Die erfindungsgemäßen Mikroemulsionen sind im Gegensatz zu Emulsionen thermodynamisch stabile, optisch transparente und makroskopisch homogene Mischungen aus zwei nicht miteinander mischbaren Flüssigkeiten, nämlich von Wasser (B) und einer Ölkomponente (C), denen die unter (A) erwähnten Tensidmoleküle zugesetzt wurden. Die erf'mdungsgemäßen Mikroemulsionen sind z.B. bei Temperaturen von 20 bis 80°C, vorzugsweise unter 55°C, herstellbar und bis zu einer Temperatur von 60 °C stabil. Die mittlere Teilchengröße der dispersen Phase beträgt vorzugsweise weniger als 100 nm.

Die beanspruchten Mikroemulsionen weisen in der Regel über einen breiten Zusammensetzungsbereich keine Bildung von flüssigkristallinen Phasen auf. Vorteilhaft finden die beanspruchten Mikroemulsionen Verwendung im kosmetischen Bereich und/oder zur Herstellung eines Arzneimittels zur Verwendung im medizinisch-dermatologischen Bereich. Besonders bevorzugt werden die erfindungsgemäßen Mikroemulsionen als oder in Körperreinigungs- und Körperpflegemitteln eingesetzt.

Die Mikroemulsionen im Sinn der vorliegenden Erfindung stellen einfach herzustellende und kostengünstige Zubereitungen dar. Sie weisen gleichzeitig eine gute Schaumentwicklung und eine hohe Reinigungskraft auf. Aufgrund des Ölgehalts wirken diese Mikroemulsionen regenerierend in Bezug auf den allgemeinen Hautzustand, vermindern das Trockenheitsgefühl der Haut und machen die Haut geschmeidig.

Besonders vorteilhaft enthalten die erfindungsgemäßen Zusammensetzungen Alkanoiammoniumsalze der Alkylsulfate und/oder Alkylpolyalkylenglykolethersulfate der oben beschriebenen allgemeinen Struktur, die unabhängig von einander vorzugsweise die folgenden Reste aufweisen:
- R¹ =: C₁₂- bis G₁₆- Alkyl, wobei der Alkylrest linear und gesättigt ist,
- p =: 2 oder 3 ist, wobei p für jedes m verschieden sein kann,
- R² =: H oder Hydroxyisopropyl,
- R³ =: H oder Hydroxyisopropyl,
- R⁴ =: Hydroxyisopropyl und
- m =: 0 , 1 oder 2 ist.

Im folgenden werden vorteilhafte Ausführungsformen der Erfindung in Bezug auf die Komponenten (C) bis (G) erläutert.

### Ölkomponente (C)

Vorteilhaft werden erfindungsgemäße Ölkomponenten aus der Gruppe der Lecithine, und der Mono-, Di- und /oder Triglyceride gesättigter und/oder ungesättigter, verzweigter und/oder linearer Alkylcarbonsäuren einer Kettenlänge von 8 bis 24, insbesondere 12 bis 18 C-Atomen, eingesetzt. Die Fettsäuretriglyceride können vorteilhaft synthetische, halbsynthetische oder natürliche Öle sein, wie z.B. Sojaöl, Rizinusöl, Olivenöl, Safloröl, Weizenkeimöl, Traubenkernöl, Sonnenblumenöl, Erdnußöl, Mandelöl, Palmöl, Kokosöl, Distelöl, Nachtkerzenöl, Rapsöl und dergleichen.

Weiterhin kann die Ölkomponente Vaseline, Paraffinöl und Polyolefine enthalten oder aus diesen bestehen. Die Ölkomponenten können im Sinne der vorliegenden Erfindung ferner vorteilhaft gewählt werden aus der Gruppe der Ester aus gesättigten und/oder ungesättigten, verzweigten und/oder linearen Alkylcarbonsäuren einer Kettenlänge von 3 bis 30 C-Atomen und gesättigten und/oder ungesättigten, verzweigten und/oder linearen Alkoholen einer Kettenlänge von 3 bis 30 C-Atomen sowie aus der Gruppe der Ester aus aromatischen Carbonsäuren und gesättigten und/oder ungesättigten, verzweigten und/oder linearen Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat, sowie synthetische, halbsynthetische und natürliche Gemische solcher Ester, wie z.B. Jojobaöl.

Ferner kann die Ölkomponente vorteilhaft gewählt werden aus der Gruppe der verzweigten und linearen Kohlenwasserstoffe und -wachse und der Silikonöle. Auch beliebige Mischungen der vorgenannten Ölkomponenten sind vorteilhaft im Sinne der Erfindung.

### Alkohole (D)

Die beanspruchten Mikroemulsionen enthalten ein- oder mehrwertige, vorzugsweise ein-, zwei- oder dreiwertige, C₂- bis C₂₄- Alkohole, vorzugsweise gesättigte und/oder verzweigte und/oder lineare Alkohole. Beispielhaft seien genannt: Ethanol, Propanol, Isopropanol, Butanol, Pentanol, Hexanol, Heptanol, Oktanol, 2-Ethylhexanol, Laurylalkohol, Myristylalkohol, Palmitylalkohol, Sterylalkohol, Oleylalkohol, Elaidylalkohol, Guerbetalkohole und Alkylenglykole, wie z.B. Ethylenglykol, Propylenglykol und Glycerin. Besonders bevorzugt ist Propylenglykol.

### Weitere Tenside (E)

Neben den genannten Alkanolammoniumsalzen der Alkylsulfate und/oder Alkylpolyalkylenglykolethersulfate können die erfindungsgemäßen Mikroemulsionen weitere Tenside enthalten. Vorteilhaft werden diese gewählt aus der Gruppe der
- Alkoholpolyethylenglykolether, z.B. solchen der allgemeinen Formel R-O-(C₂H₄O)ₙ-H, wobei R einen verzweigten oder linearen, gesättigten oder ungesättigten C₈- bis C₂₀- Alkylrest und n eine Zahl von 2 bis 20 darstellen, Fettsäureesterpolyethylenglykolether, z.B. solchen der allgemeinen Formel R-COO-(C₂H₄O)ₚ-H, wobei R einen verzweigten oder linearen, gesättigten oder ungesättigten C₇- bis C₁₉- Alkylrest und p eine Zahl von 2 bis 40 darstellen,
- Alkylpolyalkylenglykolethercarbonsäuren, z.B. solchen der allgemeinen Formel R-O-(C₂H₄O)ₙ-CH₂-COOH bzw. deren Alkanolammonium- oder Alkalimetallsalze, wobei R einen verzweigten oder linearen, gesättigten oder ungesättigten C₈- bis C₂₀- Alkylrest und n eine Zahl von 2 bis 20 darstellen,
- Alkylamidoalkylbetaine, z.B. solchen der allgemeinen Formel R-CONH(CH₂)ᵤN⁺(CH₃)₂- CH₂-COO⁻, wobei R einen verzweigten oder linearen, gesättigten oder ungesättigten C₇- bis C₁₉- Alkylrest und u eine Zahl von 1 bis 10 darstellen,
- Produkte aus der Alkoxylierung von Triglyceriden, die ganz oder teilweise mit C₆- bis C₂₂- Fettsäuren verestert sind, wobei pro Mol Triglycerid 2 bis 40 Mol Alkoxylierungsmittel eingesetzt werden, z.B. mit Ölsäure teilveresterte Anlagerungsprodukte von Rizinusöl und/oder gehärtetem Rizinusöl mit Ethylenoxid. Vorzugsweise sind in den erfindungsgemäßen Mikroemulsionen keine oder allenfalls sehr geringe Anteile (kleiner 1,5 Gew.-%) Polyhydroxyfettsäureamide, sogenannte Glucamide, beigefügt. Vorzugsweise sind in der erfindungsgemäßen Zusammensetzung weiterhin keine oder allenfalls sehr geringe Anteile (kleiner 0,5 Gew.-%) anionischen Tenside des Sulphonat-Typs enthalten.

### Elektrolyte (F)

Die erfindungsgemäßen Mikroemulsionen können Elektrolyte enthalten. Beispielhaft seien Alkali- und Erdalkalisalze, wie z.B. die entsprechenden Halogenide, Sulfate, Phosphate oder Citrate genannt.

### Additive (G)

Additive sind beispielhaft Poly(C₂- bis C₄- )alkylenglykole, insbesondere Polyethylenglykole und/oder Polypropylenglykole, jeweils vorzugsweise mit einem Molgewicht bis 1500 g/mol, Parfüme, Farbstoffe, Hydrotropica, Verdicker, Perlglanzagenzien, Eiweißhydrolysate, Pflanzenextrakte, Vitamine, antimikrobielle Stoffe und dergleichen.

Die nachfolgenden Beispiele, sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Mikroemulsionen.

| Beispiel 1: | |
|---|---|
| MARLINAT® 242/90 M | 25% |
| MARLIPAL® 24/99 | 9 % |
| Paraffinöl | 5% |
| NaCl | 8% |
| Parfüm, Antioxidans, Konservierungsstoff | q.s. |
| Wasser | Rest zu 100% |
| Herstellung: Die drei erstgenannten Komponenten werden bei 80°C homogen vermischt und bei der gleichen Temperatur mit wässriger NaCl-Lösung versetzt. Parfüm, Antioxidans und Konservierungsstoff werden bei 30°C zugesetzt. | |

| Beispiel 2: | |
|---|---|
| MARLINAT ® 242/90 M | 30 % |
| n-Hexanol | 4% |
| Paraffinöl | 5 % |
| NaCl | 4 % |
| Parfüm, Antioxidans, Konservierungsstoff | q.s. |
| Wasser | Rest zu 100 % |
| Herstellung: Wie Beispiel 1 | |

| Beispiel 3: | |
|---|---|
| MARLINAT ® 242/90 M | 38 % |
| Paraffinöl | 5% |
| NaCl | 5% |
| Parfüm, Antioxidans, Konservierungsstoff | q.s. |
| Wasser | Rest zu 100% |
| Herstellung: Die zwei erstgenannten Komponenten werden bei 80°C homogen vermischt und bei der gleichen Temperatur mit wässriger NaCl-Lösung versetzt. Parfüm, Antioxidans und Konservierungsstoff werden bei 30°C zugesetzt | |

| Beispiel 4: | |
|---|---|
| MARLINAT® 242/90 M | 28 % |
| MARLIPAL® 24/99 | 9 % |
| Paraffinöl | 5% |
| Ampholyt JB 130 K | 9% |
| NaCl | 8% |
| Parfüm, Antioxidans, Konservierungsstoff | q.s. |
| Wasser | Rest zu 100% |
| Herstellung: Die drei erstgenannten Komponenten werden bei 80°C homogen vermischt und bei der gleichen Temperatur mit wässriger NaCl-Lösung und der Komponente 4 versetzt. Parfüm, Antioxidans und Konservierungsstoff werden bei 30°C zugesetzt. | |

| Beispiel 5: | |
|---|---|
| MARLINAT® 242/90 M | 28 % |
| MARLIPAL® 24/99 | 9 % |
| MARLINAT® CM 105/80 | 5 % |
| Paraffinöl | 5% |
| NaCl | 8% |
| Parfüm, Antioxidans, Konservierungsstoff | q.s. |
| Wasser | Rest zu 100 % |
| Herstellung: Die vier erstgenannten Komponenten werden bei 80°C homogen vermischt und bei der gleichen Temperatur mit wässriger NaCl-Lösung versetzt. Parfüm, Antioxidans und Konservierungsstoff werden bei 30°C zugesetzt. | |

| Beispiel 6: | |
|---|---|
| MARLINAT® 242/90 M | 30 % |
| MARLIPAL® 24/70 | 15 % |
| Sojaöl | 5% |
| NaCl | 4% |
| Parfüm, Antioxidans, Konservierungsstoff | q.s. |
| Wasser | Rest zu 100% |
| Herstellung: Wie Beispiel 1 | |

| Beispiel 7: | |
|---|---|
| MARLINAT® 242/90 M | 30 % |
| MARLIPAL® 24/70 | 10 % |
| Paraffinöl | 5% |
| Na-citrat | 4% |
| Parfüm, Antioxidans, Konservierungsstoff | q.s. |
| Wasser | Rest zu 100% |
| Herstellung: Wie Beispiel 1, anstelle der wässrigen NaCl-Lösung wird eine wässrige Na-citrat-Lösung verwendet. | |

| Beispiel 8: | |
|---|---|
| MARLINAT® 242/90 T | 30 % |
| MARLIPAL® 24/60 | 10% |
| Paraffinöl | 5% |
| NaCl | 7% |
| Parfüm, Antioxidans, Konservierungsstoff | q.s. |
| Wasser | Rest zu 100 % |
| Herstellung: Die drei erstgenannten Komponenten werden bei 50°C homogen vermischt und bei der gleichen Temperatur mit wässriger NaCl-Lösung versetzt. Parfüm, Antioxidans und Konservierungsstoff werden bei 30°C zugesetzt. | |

| Beispiel 9: | |
|---|---|
| MARLINAT® 242/90 M | 28 % |
| LIPOXOL® 600 | 2% |
| MARLOWET®LVS | 7% |
| Sojaöl | 4% |
| Rizinusöl | 1% |
| MARLINAT® CM 105/80 | 4 % |
| Ampholyt JB 130 K | 5% |
| NaCl | 2% |
| Parfüm, Eiweißhydrolysat, Verdicker, | |
| Antioxidans, Konservierungsstoff | q.s. |
| Wasser | Rest zu 100% |
| Herstellung: Die 6 erstgenannten Komponenten werden bei 20°C homogen vermischt und bei der gleichen Temperatur mit den übrigen Komponenten versetzt. | |

| Beispiel 10: | |
|---|---|
| MARLINAT® 242/90 M | 30 % |
| LIPOXOL® 600 | 2% |
| MARLOWET® LVS | 5% |
| Sojaöl | 2% |
| Paraffinöl | 3% |
| MARLINAT® CM 105/80 | 4 % |
| Ampholyt JB 130 K | 5% |
| NaCl | 2% |
| Parfüm, Eiweißhydrolysat, Verdicker, | |
| Antioxidans, Konservierungsstoff | q.s. |
| Wasser | Rest zu 100% |
| Herstellung: Wie Beispiel 9. | |

In den Versuchsbeispielen 1 bis 10 wurden die folgenden Produkte der CONDEA Chemie GmbH eingesetzt:

| | |
|---|---|
| MARLINAT® 242/90 M | 90 % C₁₂- bis C₁₄- Alkylpolyethylenglykol (2 EO) ethersulfat - Monoisopropanolammonium (MIPA) Salz, in 1,2-Propylenglykol, |
| MARLINAT® 242/90 T | 90 % C₁₂- bis C₁₄- Alkylpolyethylenglykol (2 EO) ethersulfat- Triisopropanolammonium (TIPA) Salz in 1,2-Propylenglykol, |
| MARLIPAL® 24/60 | C₁₂- bis C₁₄- Fettalkoholpolyethylenglykol (6 EO) ether, |
| MARLIPAL® 24/70 | C₁₂- bis C₁₄- Fettalkoholpolyethylenglykol (7 EO) ether, |
| MARLIPAL® 24/99 | 90 % C₁₂- bis C₁₄- Fettalkoholpolyethylenglykol (9 EO) ether, in Wasser, |
| MARLINAT® CM 105/80 | 80 % C₁₂- bis C₁₄- Alkylpolyethylenglykol (10 EO) ethercarbonsäure-Natriumsalz in Wasser, |
| MARLOWET® LVS | Oxethyliertes Rizinusöl, teilverestert mit Ölsäure |
| LIPOXOL® 600 | Polyethylenglykol 600, |
| Ampholyt JB 130 K | 30 % Kokoamidopropyldimethylbetain in Wasser. |

Alle beispielhaft angegebenen Formulierungen zeichnen sich durch hohe Reinigungskraft, hohes Schäumvermögen, gutes Anschäumvermögen, Lagerstabilität und Hautmildheit aus.

## Patentansprüche

1. Mikroemulsion enthaltend zumindest die folgenden Komponenten:
(A) 0,5 bis 70 Gew.-% Alkanolammonium-Salze der Alkylsulfate und/oder Alkylpolyalkylenglykolethersulfate der Struktur:
R¹-O-(CₚH₂ₚO)ₘ-SO₃⁻ HN⁺R²R³R⁴ ,
worin
R¹ = ein C₈- bis C₂₀- Kohlenwasserstoffrest ist,
p = eine ganze Zahl von 2 bis 5 ist, wobei p für jedes m verschieden sein kann,
R² = H, ein C₁- bis C₆- Alkyl oder ein Hydroxyisopropyl,
R³ = H, ein C₁- bis C₆- Alkyl oder ein Hydroxyisopropyl,
R⁴ = Hydroxyisopropyl und
m = eine ganze Zahl von 0 bis 7 ist,
oder deren Gemische,
(B) 20 bis 95 Gew.-% Wasser und
(C) 0,1 bis 20 Gew.-% eine oder mehrere Ölkomponenten und
(D) 0,1 bis 20 Gew.-% ein oder mehrere ein- oder mehrwertige C₂- bis C₂₄- Alkohole,
jeweils bezogen auf die Gesamtzusammensetzung.

2. Mikroemulsion gemäß Anspruch 1, worin die Alkanolammonium-Salze der Alkylsulfate und/oder Alkylpolyalkylenglykolethersulfate folgende Reste bzw. Indizes aufweisen:
R¹ = ein linearer oder gesättigter C₁₂- bis C₁₆- Alkylrest,
p = 2 oder 3 ist, wobei p für jedes m verschieden sein kann,
R² = H oder Hydroxyisopropyl,
R³ = H oder Hydroxyisopropyl,
R⁴ = Hydroxyisopropyl und
m = eine ganze Zahl von 0 bis 2 ist.

3. Mikroemulsion gemäß einem der vorhergehenden Ansprüche, wobei die Komponenten
(A) zu 2 bis 60, Gew.-%,
(B) zu 30 bis 80 Gew.-%,
(C) zu 0,5 bis 15 Gew.-% und
(D) zu 0,1 bis 9 Gew.-%
in der Mikroemulsion enthalten sind.

4. Mikroemulsion gemäß einem der vorhergehenden Ansprüche, weiterhin enthaltend zumindest eine der folgenden Komponenten
(E) 0 bis 20 Gew.-% eines oder mehrerer weiterer Tenside,
(F) 0 bis 20 Gew.-% eines oder mehrerer Elektrolyte und
(G) 0 bis 10 Gew.-% eines oder mehrerer Additive .

5. Mikroemulsion gemäß Anspruch 4, enthaltend zumindest eine der folgenden Komponenten
(E) als zumindest ein weiteres Tensid mit Ethylenoxid und/oder Propylenoxid oxalkylierte und anschließend mit C₆- bis C₂₂- Fettsäuren ganz oder teilweise veresterte Triglyceride und
(G) als zumindest ein Additiv Poly(C₂- bis C₄-)alkylenglykole mit einem Molgewicht von bis zu 1500 g/mol.

6. Mikroemulsion gemäß einem der vorhergehenden Ansprüche, wobei die Ölkomponente (C) eine oder mehrere Komponenten enthält ausgewählt aus der Gruppe der Lecithine; der Mono-, Di- und /oder Triglyceride gesättigter und/oder ungesättigter, verzweigter und/oder linearer Carbonsäuren einer Kettenlänge von 8 bis 24 C-Atomen; verzweigten und/oder linearen Kohlenwasserstoffen; Wachse; Vaseline; Paraffinöle; Polyolefine; Silikonöle und Ester aus gesättigten, ungesättigten und/oder aromatischen, verzweigten und/oder linearen Carbonsäuren einer Kettenlänge von 3 bis 30 C-Atomen und gesättigter und/oder ungesättigter, verzweigter und/oder linearer Alkohole einer Kettenlänge von 3 bis 30 C-Atomen.

7. Mikroemulsion gemäß einem der vorhergehenden Ansprüche, **dadurch gekennzeichnet, daß** die Mikroemulsion eine stabile und transparente Emulsion ist, deren disperse Phase eine mittlere Teilchengröße von kleiner 100 nm aufweist.

8. Verwendung der Mikroemulsion gemäß einem der vorhergehenden Ansprüche im kosmetischen Bereich.

9. Verwendung der Mikroemulsion gemäß einem der Ansprüche 1-7 zur Herstellung eines Arzneimittels zur Verwendung im medizinisch-dermatologischen Bereich.

10. Verwendung der Mikroemulsion gemäß einem der Ansprüche 1 bis 7 als Körperreinigungs- und/oder Körperpflegemittel.

11. Verwendung der Mikroemulsion gemäß Anspruch 10 zur Reinigung und Pflege der Haare, **dadurch gekennzeichnet, daß** die Komponente
(C) zu 0,1 bis 2 Gew.-%
in der Mikroemulsion enthalten ist.

## Claims

1. A microemulsion containing at least the following components:
(A) 0.5 to 70 % by weight alkanolammonium salts of the alkylsulfates and/or alkylpolyalkyleneglycolethersulfates having the structure:
R¹-O-(CₚH₂ₚO)ₘ-SO₃⁻HN⁺R²R³R⁴ ,
wherein
R¹ = is a C₈- to C₂₀-hydrocarbon residue,
p = is an integer from 2 to 5, wherein p can be different for each m,
R² = H, a C₁- to C₆-alkyl, or a hydroxyisopropyl,
R³ = H, a C₁- to C₆-alkyl, or a hydroxyisopropyl,
R⁴ = hydroxyisopropyl, and
m = is an integer from 0 to 7,
or mixtures thereof
(B) 20 to 95 % by weight water, and
(C) 0.1 to 20 % by weight one or more oil component(s), and
(D) 0.1 to 20 % by weight of one or more mono- or polyvalent C₂- to C₂₄-alcohol(s),
each based on the total composition.

2. Microemulsion according to claim 1, wherein the alkanolammonium salts of the alkylsulfates and/or alkylpolyalkyleneglycolethersulfates comprise the following residue or indices:
R¹ = a linear or saturated C₁₂- to C₁₆-alkyl residue,
p = 2 or 3, wherein p can be different for each m,
R² = H or hydroxyisopropyl,
R³ = H or hydroxyisopropyl,
R⁴ = hydroxyisopropyl, and
m = is an integer from 0 to 2.

3. Microemulsion according to any one of the preceding claims, wherein the microemulsion contains component
(A) with 2 to 60 % by weight
(B) with 30 to 80 % by weight
(C) with 0.5 to 15 % by weight
(D) with 0.1 to 9 % by weight

4. Microemulsion according to any one of the preceding claims, further containing at least one of the following components:
(E) 0 to 20 % by weight of one or more additional surfactant(s)
(F) 0 to 20 % by weight of one or more electrolyte(s), and
(G) 0 to 10 % by weight of one or more additive(s).

5. Microemulsion according to claim 4 containing at least one of the following components
(E) as at least one additional surfactant, triglycerides alkoxylated with ethyleneoxide and/or propyleneoxide and subsequently esterified, wholly or in part, with C₆- to C₂₂-fatty acids, and
(G) as at least one additive, poly(C₂- to C₄-) alkyleneglycols having a molecular weight of up to 1,500 g/mole.

6. Microemulsion according to any one of the preceding claims, wherein the oil component (C) contains one or more components selected from the group of lecithins; of mono-, di-, and/or triglycerides of saturated and/or unsaturated, branched and/or linear carboxylic acids having chain lengths of from 8 to 24 carbon atoms; branched and/or linear hydrocarbons; waxes; vaselines; paraffin oils; polyolefins; silicone oils and esters of saturated, unsaturated, and/or aromatic, branched, and/or linear carboxylic acids having chain lengths of from 3 to 30 carbon atoms and of saturated and/or unsaturated, branched and/or linear alcohols having chain lengths of from 3 to 30 carbon atoms.

7. Microemulsion according to any one of the preceding claims, **characterized in that** the microemulsion is a stable and transparent emulsion the disperse phase thereof having an average particle size of less than 100 nm.

8. The use of the microemulsion according to any one of the preceding claims for cosmetic applications.

9. The use of the microemulsion according to any one of the preceding claims for the manufacture of a medicment for medical-dermatologic applications.

10. The use of the microemulsion according to any one of claims 1 to 7 as body cleaning and/or body care preparations.

11. The use of the microemulsion according to claim 10 for cleaning and treating hair, **characterized in that** the microemulsion contains component (C) with 0.1 to 2 % by weight.

## Revendications

1. Microémulsion contenant au moins les composants suivants :
(A) 0,5 à 70% en poids de sels d'alcanolammonium des sulfates d'alkyle et/ou des polyalkylèneglycoléthersulfates d'alkyle de structure :
R¹-O- (CₚH₂ₚO)ₘ-SO₃-HN⁺R²R³R⁴
dans laquelle
R¹ = un radical hydrocarboné en C₈ à C₂₀,
p = un nombre entier de 2 à 5, p pouvant être différent pour chaque m,
R² = H, un alkyle en C₁ à C₆ ou un hydroxyisopropyle,
R³ = H, un alkyle en C₁ à C₆ ou un hydroxyisopropyle,
R⁴ = hydroxyisopropyle et
m = un nombre entier de 0 à 7
ou leurs mélanges
(B) 20 à 95% en poids d'eau et
(C) 0,1 à 20% en poids d'un ou de plusieurs composants huileux et
(D) 0,1 à 20% en poids d'un ou de plusieurs alcools en C₂ à C₂₄ monovalents ou polyvalents,
à chaque fois par rapport à la composition totale.

2. Microémulsion selon la revendication 1, dans laquelle les sels d'alcanolammonium des sulfates d'alkyle et/ou des polyalkylèneglycoléthersulfates d'alkyle présentent les radicaux ou, selon le cas, indices suivants :
R¹ = un radical alkyle en C₁₂ à C₁₆ linéaire ou saturé
p = 2 ou 3, p pouvant être différent pour chaque m,
R² = H ou hydroxyisopropyle,
R³ = H ou hydroxyisopropyle,
R⁴ = hydroxyisopropyle et
m = un nombre entier de 0 à 2

3. Microémulsion selon l'une quelconque des revendications précédentes, les composants
(A) étant contenus à raison de 2 à 60% en poids,
(B) étant contenus à raison de 30 à 80% en poids,
(C) étant contenus à raison de 0,5 à 15% en poids,
(D) étant contenus à raison de 0,1 à 9% en poids,
dans la microémulsion.

4. Microémulsion selon l'une quelconque des revendications précédentes, contenant par ailleurs au moins l'un des composants suivants :
(E) 0 à 20% en poids d'un ou de plusieurs agents tensioactifs
(F) 0 à 20% en poids d'un ou de plusieurs électrolytes et
(G) 0 à 10% en poids d'un ou de plusieurs additifs.

5. Microémulsion selon la revendication 4, contenant au moins l'un des composants suivants :
(E) comme au moins un autre agent tensioactif des triglycérides oxalkylés avec de l'oxyde d'éthylène et/ou de l'oxyde de propylène et ensuite estérifiés totalement ou partiellement avec des acides gras en C₆ à C₂₂ et
(G) comme au moins un additif des poly(alkylène en C₂ à C₄)glycols présentant un poids moléculaire allant jusqu'à 1500 g/mole.

6. Microémulsion selon l'une quelconque des revendications précédentes, le composant huileux (C) contenant un ou plusieurs composants choisis parmi le groupe de la lécithine ; des monoglycérides, des diglycérides et/ou des triglycérides d'acides carboxyliques saturés et/ou insaturés, ramifiés et/ou linéaires présentant une longueur de chaîne de 8 à 24 atomes de carbone ; des hydrocarbures ramifiés et/ou linéaires ; des cires ; de la vaseline ; des huiles de paraffine ; des polyoléfines ; des huiles de silicone et des esters d'acides carboxyliques saturés, insaturés et/ou aromatiques, ramifiés et/ou linéaires d'une longueur de chaîne de 3 à 30 atomes de carbone et/ou d'alcools saturés et/ou insaturés, ramifiés et/ou linéaires présentant une longueur de chaîne de 3 à 30 atomes de carbone.

7. Microémulsion selon l'une quelconque des revendications précédentes, **caractérisée en ce que** la microémulsion est une émulsion stable et transparente, dont la phase dispersée présente une grosseur moyenne des particules inférieure à 100 nm.

8. Utilisation de la microémulsion selon l'une quelconque des revendications précédentes dans le domaine cosmétique.

9. Utilisation de la microémulsion selon l'une quelconque des revendications 1 à 7 pour la préparation d'un médicament pour une utilisation dans le domaine médico-dermatologique.

10. Utilisation de la microémulsion selon l'une quelconque des revendications 1 à 7 comme produit de nettoyage corporel et/ou de soin corporel.

11. Utilisation de la microémulsion selon la revendication 10 pour le nettoyage et le soin des cheveux, **caractérisée en ce que** le composant (C) est contenu à raison de 0,1 à 2% en poids dans la microémulsion.
